# EUROPEAN PATENT SPECIFICATION

(11) **EP 3 982 815 B1**
(45) Date of publication and mention of the grant of the patent: **04.11.2026**
(21) Application number: 20821807.3
(22) Date of filing: 04.06.2020
(51) Int. Cl.: A61B 1/00, A61B 1/002, G02B 23/24

(54) **METHOD OF ASSEMBLING ENDOSCOPE OPTICAL COMPONENTS**
VERFAHREN ZUR MONTAGE OPTISCHER ENDOSKOPKOMPONENTEN
PROCÉDÉ D'ASSEMBLAGE DE COMPOSANTS OPTIQUES D'ENDOSCOPE

(30) Priority: 11.06.2019 US 201916438152
(43) Date of publication of application: 20.04.2022
(60) Divisional application: 26202414.4 / 4 826 431
(73) Proprietor: Steris Instrument Management Services, Inc., Birmingham, AL 35222 (US)
(72) Inventor: YE, Shusheng, Cooper City, FL 33330 (US); BODOR, Zoltan A., Cooper City, FL 33330 (US)
(74) Representative: Bryers Intellectual Property Ltd
(86) International application number: PCT/US2020/036016
(87) International publication number: WO 2020/251823

(56) References cited:
- DE-A1- 102008 056 830
- US-A1- 2018 228 346
- US-A1- 2019 059 709
- US-B1- 6 240 231
- US-B1- 8 819 913
- US-B1- 8 819 913
- US-B2- 7 530 945
- US-B2- 7 530 945

## Description

### Cross Reference To Related Applications

This application claims priority to U.S. Non-Provisional Patent Application No. 16/438,152, entitled "Method of Assembling Endoscope Optical Components," filed on June 11, 2019.

### Technical Field

The present invention is directed to a method of assembling an optical relay system, and more particularly, to a method for assembling an endoscopic optical relay system within a flexible, shrinkable tube.

### Background of Invention

The assembly of optical relay systems within flexible tubes is known in the art. For example, U.S. Pat. No. 7,530,945 to Rudischhauser et al. describes a method for assembling an endoscope having an optical system with several components that are at least partially surrounded by a tube made of both a transparent and a shrunk material. The method includes introducing the optical components into the tube of transparent and shrinkable material to form a unit, followed by shrinking the shrinkable material of the tube for fixing the position of the components contained within the tube relative to one another. To ensure the proper positioning of the components relative to one another, the components are inspected through the transparent shrunk material of the shrunk tube. After proper positioning of the components is confirmed, the unit composed of the shrunk tube and the components contained therein is introduced into a tubular shaft of the endoscope.

U.S. Pat. No. 8,819,913 to Bodor et al. describes a method of assembling an optical relay system including forming a line of optical components within an optical component aligning groove of a first base member, axially aligning a cylinder with the aligning groove of the first base member, detachably coupling the cylinder to the first base member and directing the line of optical components from the optical component receiving groove into the cylinder. The cylinder, the line of optical components and a shrinkable tube which has previously been placed around the cylinder are then detached from the first base member and positioned within a cylinder receiving groove of a second base member. With axial movement of the cylinder restricted within the cylinder receiving groove, the line of optical components is pushed out the cylinder and into the shrinkable tube which slides off of the cylinder by virtue of its constriction about a distal end of the line of optical components. The line of optical components and shrinkable tube then are advanced along the cylinder receiving groove and through a heating area where the shrinkable tube is shrunk about the line of optical components. To ensure axial compression upon the line of optical components within the shrinkable tube, a tension device located upstream of the heating area applies pressure on the shrink tube as it and the line of optical components are advanced long the cylinder receiving groove thereby causing the tube to stretch axially while the shrinkable tube is heated and shrunk radially about the line of optics.

US 7 530 945 B2 discloses a method of assembling an optical relay system. A line of rod lenses and spacers is introduced into a heat shrinkable tube to form a unit, which is inserted into a groove. A plate-shape object presses the unit into the groove. Heat is applied to shrink the heat shrinkable tube.

### Summary of Invention

The present invention is directed to a method of assembling an optical relay system.

According to the invention, there is provided a method for assembling an optical relay system according to Claim 1. Further optional features are defined by dependent Claims 2 to 10.

The line of optical components may be fixed and optically aligned in the heat shrink tube by heating the heat shrink tube and line of optical components while positioned in a V-groove with an elongate weight resting on top of the heat shrink tube and the line of optical components pressed between a pair of opposing, biased plungers. In this manner, heat, radial pressure and axial pressure are simultaneously applied to the line of optical components which ensures both radial and axial alignment of the line of optical components. The plurality of optical components may be placed in the loading tube by radially-inserting the plurality of optical components into a staging portion of the loading tube. The alignment and order of the plurality of optical components may be observed in the staging portion through an opening in a sidewall of the loading tube. The staging portion may have a length that is defined by a length of an opening formed in a sidewall of the loading tube.

### Brief Description of The Drawings

FIG. 1 is a perspective view of a loading tube used in the present invention.
FIG. 2 is a sectional view of the loading tube of FIG. 1 taken along a length of the loading tube.
FIG. 3 is a perspective view of a heat shrink tube used in the present invention.
FIG. 4 is a partial sectional view of the heat shrink tube of FIG. 3 fixed at one end thereof about an optical component.
FIG. 5 is a perspective view of the loading tube of FIG. 1 showing the heat shrink tube of FIG. 4 inserted over a loading portion of the loading tube with a plurality of the optical components arranged in a line within the staging portion.
FIGS. 6A and 6B are perspective views of the loading tube of FIG. 1 showing the line of optical components of FIG. 5 being moved from the staging portion into the loading portion.
FIG. 7 is a sectional view of the loading tube of FIG. 1 taken along the length of the loading tube showing the line of optical components of FIG. 5 being moved from the staging portion into the loading portion.
FIG. 8 is a perspective view of the loading tube of FIG. 1 showing a line of optical components being deposited within the heat shrink tube of FIG. 4.
FIG. 9 is a partial sectional view of a line of optical components positioned within the heat shrink tube of FIG. 4 showing axial and radial misalignment of the optical components in the heat shrink tube.
FIG. 10 is a perspective view of the heat shrink tube and line of optical components of FIG. 9 showing a weight placed on the heat shrink tube for radially and axially aligning the optical components, with the line of optical components pressed between a pair of spring loaded plungers for removing gaps between the optical components.
FIG. 11 is a sectional view of a line of optical components fixed with a shrunk heat shrink tube with the line optical components optically aligned and arranged as a contiguous unit in the tube.

### Detailed Description of The Invention

The present invention is directed to a method for assembling an endoscopic optical relay. The method includes shrinking a first end of a heat shrink tube 10 about a first optical component, inserting a loading portion 12 of a loading tube 14 into a second end of heat shrink tube 10, radially-inserting a plurality of optical components into a staging portion 16 of loading tube 14 thereby forming a line of optical components, the staging portion being seamlessly coupled to and integrally-formed with loading portion 12, moving the line of optical components from staging portion 16 into loading portion 12, and removing loading portion 12 from between the line of optical components and heat shrink tube 10 thereby depositing the line of optical components in heat shrink tube 10. The line of optical components is fixed and optically aligned within heat shrink tube 10 by applying radial pressure, axial pressure and heat to the line of optical components simultaneously.

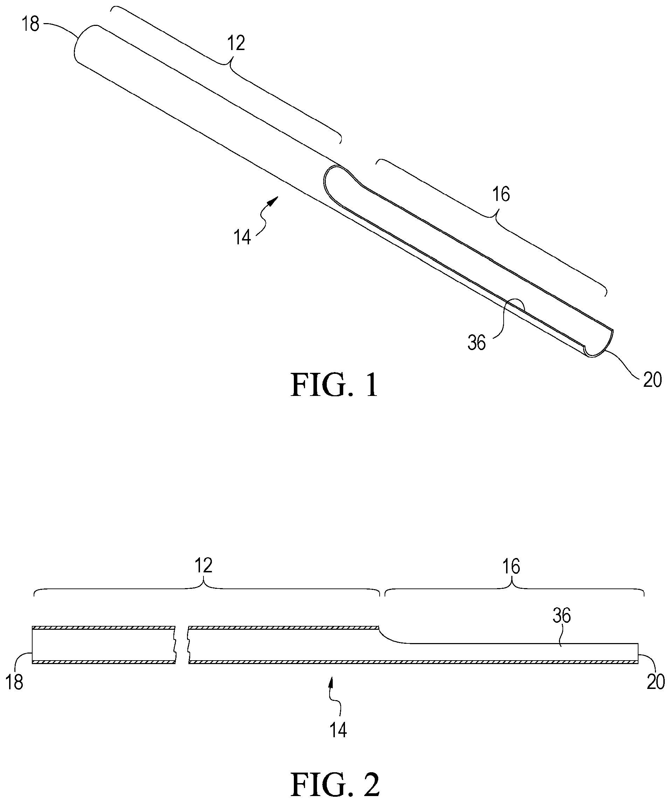

More particularly, as depicted in FIGS. 1 and 2, loading tube includes a first end 18 and an opposing second end 20. First end 18 has a first opening therethrough defined by a continuous sidewall that extends towards second end 20 to form loading portion 12. Second end 20 is formed by a semi-circular sidewall that extends towards first end 22 to form staging portion 16. The semi-circular sidewall is formed by removing a semi-circular portion from the cylinder. Staging portion 16 has a length sufficient to contain a complete endoscopic relay system. Though loading tube 14 is shown in FIGS. 1 and 2 as having a round cross-section and thus constructed from a cylinder, it is understood that the tube from which loading tube 14 is constructed may have a square, triangular, hexagonal, heptagonal, decagonal, nonagonal or other cross-section. Loading tube 14 is constructed from a seamless tube fabricated from plastic, metal, ceramic, wood, or any other suitable material.

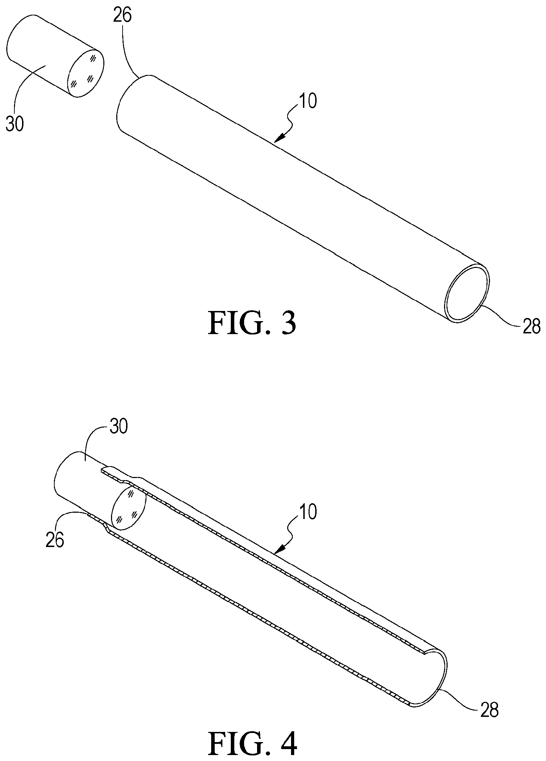

Referring to FIG. 3, heat shrink tube 10 is constructed from a thin, flexible, heat-shrinkable plastic cylinder. Heat shrink tube 10 includes a first end 26, an opposing second end 28 and a continuous sidewall extending there between. Heat shrink tube 10 has an inner diameter that is slightly greater than an outer diameter of loading portion 12 of loading tube 14.

As illustrated in FIG. 4, a first optical component 30 such as a rod lens is fixed within first end 26 of heat shrink tube 10. This occurs by manually inserting first optical component 30 partially within first end 26 and heating first end 26 using a heat source to shrink only that portion of heat shrink tube 10 that is immediately adjacent to the radial surface of first optical component 30. Although first optical component 30 is shown to partially extend out of tube 10, it is understood that first optical component 30 may be entirely enclosed within first end 26 of heat shrink tube 10.

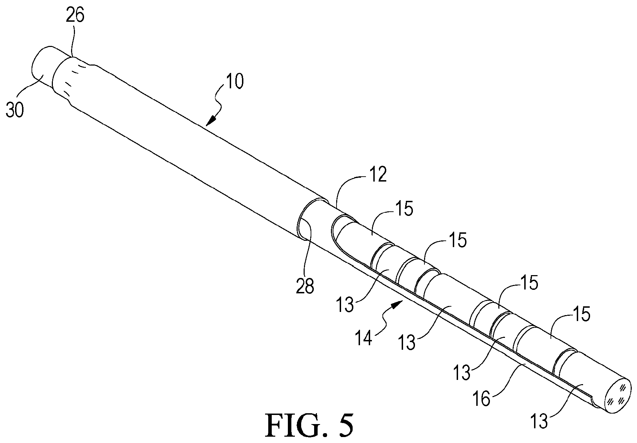

Referring to FIG. 5, after first optical component 30 is fixed within heat shrink tube 10, first end 18 of loading portion 12 of loading tube 14 is inserted through second end 28 of heat shrink tube 10 with first end 18 of loading tube 14 pressed against first optical component 30. Thereafter, a plurality of optical components, which may consist of rod lenses 13, spacers 15, aperture stops and/or other optical components, are manually placed into staging portion 16 in an order, *i.e.,* proximal to distal, required for constructing the desired optical relay assembly. Because the plurality of optical components can be placed radially into staging portion 16, there is no requirement that the components be placed into the staging portion in any particular sequence. Thus, for example, lens 13 extending out of second end 20, as depicted in FIG. 5, may be placed in staging portion 16 before the spacer 13 arranged immediately adjacent to such lens 13, since there is no requirement that the plurality of optical components be inserted axially through an end of loading tube 14.

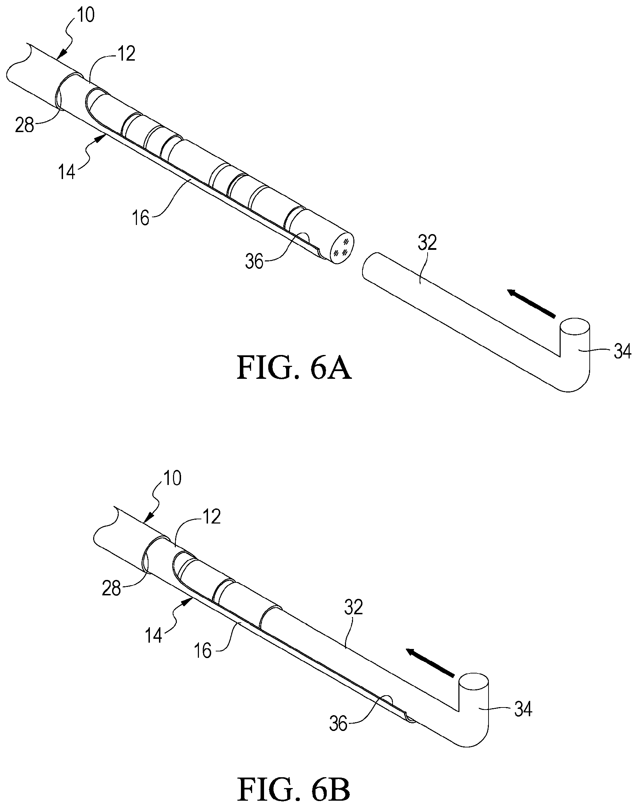

Once the plurality of optical components is in place thereby forming a line of optical components in staging portion 16, a push rod 32 is pressed against the end of the line of optical components, as shown in FIGS. 6A and 6B, towards first optical components 30 thereby forcing the line of optical components to slide as a continuous unit out of staging portion 16 and into loading portion 12. As push rod 32 pushes the line of optical components through staging portion 16 and into loading portion 12, a radially extending handle 34 of the push rod extends radially outward and travels axially through a radial opening defined by a sidewall 36 of staging portion 16.

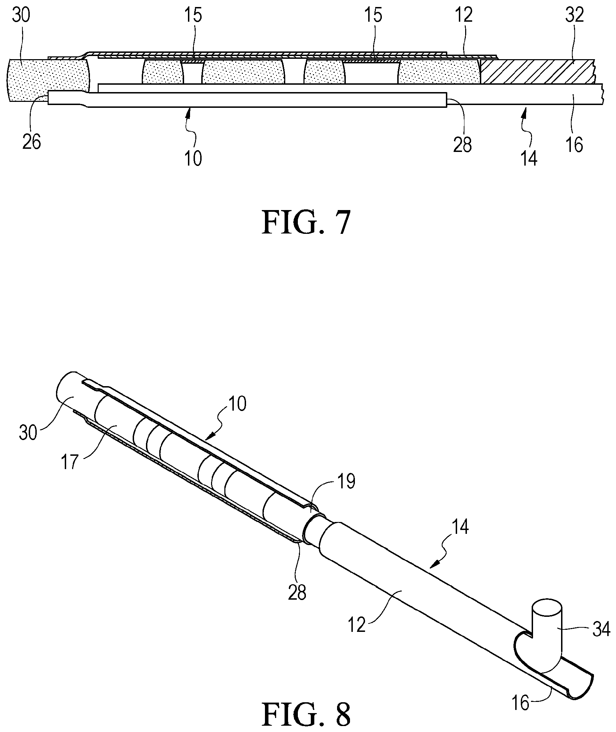

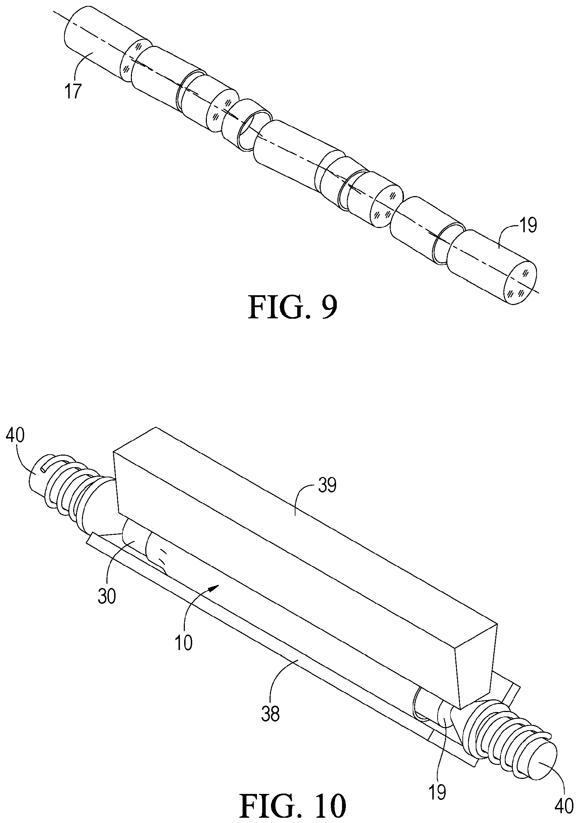

As depicted in FIG. 8, a leading optical component 17 of the line of optical components slides through loading portion 12 and eventually directly contacts first optical component 30. As the line of optical components is further pressed against first optical component 30, heat shrink tube 10 is caused to slide off loading portion 12 of loading tube 14 with the line of optical components deposited within heat shrink tube 10. This occurs with no stretching or minimal stretching of tube 10. As illustrated in FIGS. 8 and 9, the line of optical components has a length sufficient to cause a trailing optical component 19 of the line of optical component to extend out of second end 28 of heat shrink tube 10. Furthermore, the line of optical components when fully deposited within heat shrink tube 10 are axially and radially misaligned and include spaces between the various optical components.

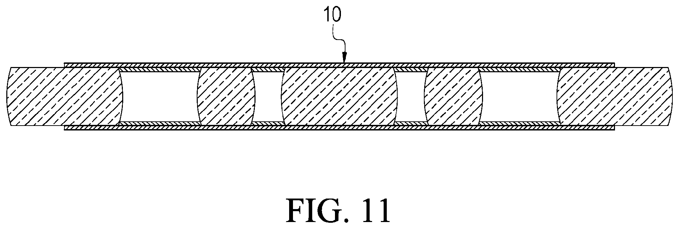

Referring to FIG. 10, to optically align the line of optical components within heat shrink tube 10, heat shrink tube 10 is placed within a V-shaped groove of an elongate base member 38 and pressure is applied axially and radially to the heat shrink tube while simultaneously heating tube 10. In particular, an elongate weight 39 is placed on a top surface of heat shrink tube 10, while the tube is in the V-groove, and a pair of opposing spring-loaded plungers 40 are arranged to press against first optical component 30 and trailing optical component 19 of the line of optical components, respectively. The pressure exerted by weight 39 on heat shrink tube 10 forces the line of optical components into radial and axial alignment, while opposing plungers 40 force the line of optical components into a contiguous unit with no spacing between the optical components. Arranged in this manner, heat shrink tube 10 and the contiguous and optically aligned line of optical components are heated, for example, in an oven. Heating causes heat shrink tube 10 to shrink both axially and radially thereby pressing the line of optical components together both axially and radially. As depicted in FIG. 11, following heating of heat shrink tube 10, the line of optical components are axially and radially aligned within tube 10 with no spaces between the optical components thereby forming a functional optical relay system for use in an endoscope.

## Claims

1. A method for assembling an optical relay system comprising:
shrinking a first end (26) of a heat shrink tube (10) about a first optical component (30),
inserting a loading portion (12) of a loading tube (14) into a second end (28) of the heat shrink tube (10),
placing a plurality of optical components (13, 15, 17, 19) in a staging portion (16) of the loading tube (14) thereby forming a line of optical components (13, 15, 17, 19), the staging portion (16) having an open radial portion,
moving the line of optical components (13, 15, 17, 19) from the staging portion (16) into the loading portion (12),
removing the loading portion (12) from between the line of optical components (13, 15, 17, 19) and the heat shrink tube (10) thereby depositing the line of optical components (13, 15, 17, 19) in the heat shrink tube (10), **characterized in that** the method comprises
fixing the line of optical components (13, 15, 17, 19) within the heat shrink tube (10) with the line of optical components (13, 15, 17, 19) optically aligned by simultaneously applying radial pressure to the line of optical components (13, 15, 17, 19), axial pressure to the line of optical components (13, 15, 17, 19) and heat.

2. The method of claim 1 including placing the heat shrink tube (1) and the line of optical components (13, 15) in a V-groove.

3. The method of claim 2 including placing an elongate weight (39) on top of the heat shrink tube (10) and the line of optical components (13, 15, 17, 19) for applying radial pressure to the line of optical components (13, 15, 17, 19).

4. The method of claim 2 including applying axial pressure to the line of optical components (13, 15, 17, 19) using a pair of opposing, biased plungers (40).

5. The method of claim 1 including shrinking the heat shrink tube (10) about the first optical element (30) by applying heat only to the first end (26) of the heat shrink tube (10).

6. The method of claim 1 including placing the plurality of optical components (13, 15, 17, 19) in the staging portion (16) of the loading tube (14) by lowering them into the staging portion (16) through the open radial portion.

7. The method of claim 1 including removing the loading tube (14) from between the line of optical components (13, 15, 17, 19) and the heat shrink tube (10) by pushing the line of optical components (13, 15, 17, 19) against the first optical component (30) and out of the loading tube (14) thereby causing the heat shrink tube (10) to slide off of the loading portion (12) of the loading tube (14).

8. The method of claim 1 wherein a portion of the first optical component (30) extends out of the heat shrink tube (10).

9. The method of claim 1 wherein the line of optical components (13, 15, 17, 19) is misaligned radially and axially and includes a gap between at least two optical components (13, 15) of the plurality of optical components (13, 15, 17, 19).

10. The method of claim 1 including fixing a second optical component (19) of the plurality of optical components (13, 15, 17, 19) in the second end (28) of the heat shrink tube (10) with a portion of the second optical component (19) extending out of the heat shrink tube (10).

## Patentansprüche

1. Verfahren zur Montage eines optischen Relaissystems, umfassend:
Schrumpfen eines ersten Endes (26) eines Schrumpfschlauchs (10) um eine erste optische Komponente (30),
Einführen eines Ladeabschnitts (12) eines Laderohrs (14) in ein zweites Ende (28) des Schrumpfschlauchs (10),
Anordnen einer Mehrzahl von optischen Komponenten (13, 15, 17, 19) in einem Bereitstellungsabschnitt (16) des Laderohrs (14), wodurch eine Reihe optischer Komponenten (13, 15, 17, 19) gebildet wird, wobei der Bereitstellungsabschnitt (16) einen offenen radialen Abschnitt aufweist,
Bewegen der Reihe optischer Komponenten (13, 15, 17, 19) von dem Bereitstellungsabschnitt (16) in den Ladeabschnitt (12),
Entfernen des Ladeabschnitts (12) aus dem Bereich zwischen der Reihe optischer Komponenten (13, 15, 17, 19) und dem Schrumpfschlauch (10), wodurch die Reihe optischer Komponenten (13, 15, 17, 19) in dem Schrumpfschlauch (10) abgelegt wird, **dadurch gekennzeichnet, dass** das Verfahren Folgendes umfasst
Fixieren der Reihe optischer Komponenten (13, 15, 17, 19) innerhalb des Schrumpfschlauchs (10), wobei die Reihe optischer Komponenten (13, 15, 17, 19) optisch ausgerichtet ist, indem gleichzeitig radialer Druck auf die Reihe optischer Komponenten (13, 15, 17, 19), axialer Druck auf die Reihe optischer Komponenten (13, 15, 17, 19) und Wärme aufgebracht werden.

2. Verfahren nach Anspruch 1, umfassend das Anordnen des Schrumpfschlauchs (1) und der Reihe optischer Komponenten (13,15) in einer V-Nut.

3. Verfahren nach Anspruch 2, umfassend das Anordnen eines länglichen Gewichts (39) oben auf dem Schrumpfschlauch (10) und der Reihe optischer Komponenten (13, 15, 17, 19) zum Aufbringen eines radialen Drucks auf die Reihe optischer Komponenten (13, 15, 17, 19).

4. Verfahren nach Anspruch 2, umfassend das Aufbringen eines axialen Drucks auf die Reihe optischer Komponenten (13, 15, 17, 19) unter Verwendung eines Paares gegenüberliegender, vorgespannter Stößel (40).

5. Verfahren nach Anspruch 1, umfassend das Schrumpfen des Schrumpfschlauchs (10) um das erste optische Element (30) durch Aufbringen von Wärme nur auf das erste Ende (26) des Schrumpfschlauchs (10).

6. Verfahren nach Anspruch 1, umfassend das Anordnen der Mehrzahl von optischen Komponenten (13, 15, 17, 19) in dem Bereitstellungsabschnitt (16) des Laderohrs (14) durch Absenken derselben in den Bereitstellungsabschnitt (16) durch den offenen radialen Abschnitt.

7. Verfahren nach Anspruch 1, umfassend das Entfernen des Laderohrs (14) aus dem Bereich zwischen der Reihe optischer Komponenten (13, 15, 17, 19) und dem Schrumpfschlauch (10) durch Drücken der Reihe optischer Komponenten (13, 15, 17, 19) gegen die erste optische Komponente (30) und aus dem Laderohr (14) heraus, wodurch bewirkt wird, dass der Schrumpfschlauch (10) von dem Ladeabschnitt (12) des Laderohrs (14) abgleitet.

8. Verfahren nach Anspruch 1, wobei sich ein Abschnitt der ersten optischen Komponente (30) aus dem Schrumpfschlauch (10) heraus erstreckt.

9. Verfahren nach Anspruch 1, wobei die Reihe optischer Komponenten (13, 15, 17, 19) radial und axial falsch ausgerichtet ist und einen Spalt zwischen mindestens zwei optischen Komponenten (13, 15) der Mehrzahl von optischen Komponenten (13, 15, 17, 19) aufweist.

10. Verfahren nach Anspruch 1, umfassend das Fixieren einer zweiten optischen Komponente (19) der Mehrzahl von optischen Komponenten (13, 15, 17, 19) in dem zweiten Ende (28) des Schrumpfschlauchs (10), wobei sich ein Abschnitt der zweiten optischen Komponente (19) aus dem Schrumpfschlauch (10) heraus erstreckt.

## Revendications

1. Procédé d'assemblage d'un système de relais optique comprenant :
le rétrécissement d'une première extrémité (26) d'un tube thermorétractable (10) autour d'un premier composant optique (30),
l'insertion d'une partie de chargement (12) d'un tube de chargement (14) dans une seconde extrémité (28) du tube thermorétractable (10),
le placement d'une pluralité de composants optiques (13, 15, 17, 19) dans une partie de préparation (16) du tube de chargement (14), formant ainsi une ligne de composants optiques (13, 15, 17, 19), la partie de préparation (16) ayant une partie radiale ouverte,
le déplacement de la ligne de composants optiques (13, 15, 17, 19) de la partie de préparation (16) vers la partie de chargement (12),
le retrait de la partie de chargement (12) située entre la ligne de composants optiques (13, 15, 17, 19) et le tube thermorétractable (10), déposant ainsi la ligne de composants optiques (13, 15, 17, 19) dans le tube thermorétractable (10), **caractérisé en ce que** le procédé comprend
la fixation de la ligne de composants optiques (13, 15, 17, 19) à l'intérieur du tube thermorétractable (10) avec la ligne de composants optiques (13, 15, 17, 19) optiquement alignée par l'application simultanée d'une pression radiale à la ligne de composants optiques (13, 15, 17, 19), d'une pression axiale à la ligne de composants optiques (13, 15, 17, 19) et de chaleur.

2. Procédé selon la revendication 1, comportant le placement du tube thermorétractable (1) et de la ligne de composants optiques (13, 15) dans une rainure en V.

3. Procédé selon la revendication 2, comportant le placement d'un poids allongé (39) sur le haut du tube thermorétractable (10) et la ligne de composants optiques (13, 15, 17, 19) pour appliquer une pression radiale à la ligne de composants optiques (13, 15, 17, 19).

4. Procédé selon la revendication 2, comportant l'application d'une pression axiale à la ligne de composants optiques (13, 15, 17, 19) à l'aide d'une paire de pistons opposés et précontraints (40).

5. Procédé selon la revendication 1, comportant le rétrécissement du tube thermorétractable (10) autour du premier élément optique (30) en appliquant de la chaleur uniquement à la première extrémité (26) du tube thermorétractable (10).

6. Procédé selon la revendication 1, comportant le placement de la pluralité de composants optiques (13, 15, 17, 19) dans la partie de préparation (16) du tube de chargement (14) en les abaissant dans la partie de préparation (16) à travers la partie radiale ouverte.

7. Procédé selon la revendication 1, comportant le retrait du tube de chargement (14) de l'espace entre la ligne de composants optiques (13, 15, 17, 19) et le tube thermorétractable (10) en poussant la ligne de composants optiques (13, 15, 17, 19) contre le premier composant optique (30) et hors du tube de chargement (14), causant ainsi le glissement du tube thermorétractable (10) hors de la partie de chargement (12) du tube de chargement (14).

8. Procédé selon la revendication 1, dans lequel une partie du premier composant optique (30) s'étend hors du tube thermorétractable (10).

9. Procédé selon la revendication 1, dans lequel la ligne de composants optiques (13, 15, 17, 19) est désalignée radialement et axialement et comporte un espace entre au moins deux composants optiques (13, 15) de la pluralité de composants optiques (13, 15, 17, 19).

10. Procédé selon la revendication 1, comportant la fixation d'un second composant optique (19) de la pluralité de composants optiques (13, 15, 17, 19) dans la seconde extrémité (28) du tube thermorétractable (10) avec une partie du second composant optique (19) s'étendant hors du tube thermorétractable (10).
